## Europäisches Patentamt

## European Patent Office

## Office européen des brevets

(19)

(11) Veröffentlichungsnummer: **0 312 797**

**A2**

(12) **EUROPÄISCHE PATENTANMELDUNG**

(21) Anmeldenummer: **88115647.5**

(22) Anmeldetag: **23.09.88**

(51) Int. Cl.⁴: **A61B 1/00**

(30) Priorität: **25.09.87 DE 3732266**

(43) Veröffentlichungstag der Anmeldung:
**26.04.89 Patentblatt 89/17**

(84) Benannte Vertragsstaaten:
**AT BE CH DE ES FR GB GR IT LI LU NL SE**

(71) Anmelder: **GMT Gesellschaft für medizinische Technik mbH**
**Holstenstrasse 2**
**D-2000 Hamburg 50(DE)**

(72) Erfinder: **Zinck, Michael Dr.**
**Klövensteenweg 151**
**D-2000 Hamburg 56(DE)**
Erfinder: **Siegel, Arnd Dr.**
**Blumenstrasse 31a**
**D-2000 Hamburg 60(DE)**

(74) Vertreter: **Heldt, Gert, Dr. Dipl.-Ing.**
**Neuer Wall 59 III**
**D-2000 Hamburg 36(DE)**

(54) **Verfahren und Vorrichtung zur Abbildung eines Knocheninnenraumes.**

(57) Das Verfahren dient zur Erzeugung einer bildlichen Darstellung eines Knocheninnenraumes. Im Bereich einer den Knocheninnenraum begrenzenden Knochenwandung wird eine Einführöffnung erzeugt und anschließend durch die Einführöffnung hindurch ein optischer Sensor in den Knochenraum eingeführt. Der optische Sensor ist vorgebbar positionierbar und überträgt optische Signale zu einer Projektionsvorrichtung. Durch die Einführöffnung hindurch kann ein Endoskop eingeführt werden. Darüber hinaus ist es möglich, den Knocheninnenraum zu beleuchten. Insbesondere ist dabei an eine Beleuchtung mit Kaltlicht gedacht.

Die Vorrichtung zur Erzeugung einer bildlichen Darstellung eines Knocheninnenraumes weist im Bereich der dem Knocheninnenraum zugewandt angeordneten Ausdehnung des Endoskops eine die Qualität der bildlichen Darstellung verbessernde Ausstattung auf. Die Ausstattung ist als Spreizvorrichtung ausgebildet, die mindestens eine im Bereich des Endoskops angeordnete Stütze aufweist. Die Stütze ist im Bereich des Endoskops schwenkbar gelagert. Es ist aber auch möglich, die Stütze teleskopierbar auszubilden.

EP 0 312 797 A2

## Verfahren und Vorrichtung zur Abbildung eines Knocheninnenraumes

Die Erfindung betrifft ein Verfahren zur Erzeugung einer bildlichen Darstellung eines Knocheninnenraumes.

Die Erfindung betrifft darüber hinaus eine Vorrichtung zur Erzeugung einer bildlichen Darstellung eines Knocheninnenraumes.

Bildliche Darstellungen eines Knocheninnenraumes werden beispielsweise benötigt, um Informationen über die Ausbildung von in den Knocheninnenraum eingebrachtem Knochenzement zu gewinnen und mit Hilfe dieser Darstellungen die Weiterentwicklung von Knochenzement gezielt zu steuern. Darstellungen des Knocheninnenraums werden aber darüber hinaus für die Bebilderung von Büchern oder bei der Herstellung von Filmen benötigt.

Bildliche Darstellungen des Knocheninnenraumes werden bis-lang so erzeugt, daß der Knochen im Bereich des darzustellenden Knocheninnenraumes geöffnet und der interessierende Bereich fotografiert oder gefilmt wird. Dieses Verfahren weist jedoch den Nachteil auf, daß zur bildlichen Darstellung unterschiedlicher Bereiche des Knocheninnenraumes mehrere Öffnungen im Bereich der Knochenwandung vorgesehen werden müssen. Darüber hinaus läßt sich eine derartige Darstellung des Knocheninnenraumes in der Regel nur bei bereits vom Körper getrennten Knochen oder bei verstorbenen Lebewesen durchführen. Die Erzeugung von bildlichen Darstellungen des Knocheninnenraumes von lebenden Menschen bereitet jedoch erhebliche Schwierigkeiten, da Beschädigungen des Knochens weitgehend vermieden bzw. auf das unbedingt erforderliche Maß begrenzt werden müssen.

Aufgabe der vorliegenden Erfindung ist es daher ein Verfahren der einleitend genannten Art derart zu verbessern, daß eine Abbildung mindestens eines vorgebbaren Bereiches des Knocheninnenraumes unter Berücksichtigung eines vorgebbaren Blickwinkels bei weitgehender Schonung der Knochen wandung erzeugt wird.

Diese Aufgabe wird erfindungsgemäß dadurch gelöst, daß im Bereich einer den Knocheninnenraum begrenzenden Knochenwandung eine Einführöffnung erzeugt und anschließend durch die Einführöffnung hindurch ein optische Signale zu einer Projektionsvorrichtung übertragender vorgebbar positionierbarer optischer Sensor in den Knocheninnenraum eingeführt wird.

Mit Hilfe des erfindungsgemäßen Verfahrens ist es möglich, bei Verwendung einer einzigen Einführöffnung im Bereich der Knochenwandung bildliche Darstellungen von beliebigen Bereichen des Knocheninnenraumes zu erzeugen.

Mit Hilfe des optischen Sensors kann beispielsweise ein während einer zurückliegenden Operation in den Bereich des Knocheninnenraumes eingebrachter Zement untersucht und Aufschlüsse über seine Beschaffenheit gewonnen werden. Mit Hilfe dieser Ergebnisse ist es dann möglich, Knochenzementsubstanzen gezielt weiterzuentwickeln.

Es ist darüber hinaus möglich, die bildlichen Darstellungen des Knocheninnenraumes sowie der Knochenwandungen, ihrer Defekte und Veränderungen vor, während und nach einer Operation zu erzeugen und damit den Operationsverlauf zu dokumentieren. Eine derartige bildliche Dokumentation kann beispielsweise zur Bebilderung von Büchern oder Aufsätzen verwendet werden, es ist aber auch möglich, mit Hilfe derartiger Aufzeichnungen den Verlauf einer medizinischen Behandlung auch nach einem langen Zeitraum nachzuvollziehen.

Gemäß einer bevorzugten Ausführungsform der Erfindung wird durch die Einführöffnung hindurch ein Endoskop eingeführt. Die Einführung eines Endoskopes weist den Vorteil auf, daß der Arzt, der mit der Herstellung der bildlichen Darstellung des Knocheninnenraumes beauftragt ist, mit der Handhabung von Endoskopen vertraut ist und eine große Sicherheit bei der Benutzung derartiger Vorrichtungen besitzt. Endoskope weisen darüber hinaus die für eine Verwendung in einem Knocheninnenraum erforderliche Sterilität und Kompaktheit auf. Darüber hinaus sind unbeabsichtigte Beschädigungen des Kno cheninnenraumes oder der dem Knocheninnenraum zugewandten Begrenzung der Knochenwandung bei der Verwendung derartiger Vorrichtungen durch geübte Bediener mit hoher Wahrscheinlichkeit ausgeschlossen.

Mit Hilfe der bislang bekanntgewordene Endoskope können nicht sämtliche Vorteile des erfindungsgemäßen Verfahrens optimal ausgenutzt werden. Insbesondere treten dann Probleme auf, wenn das Endoskop entlang der Wandungen des Knocheninnenraumes bewegt wird, bzw. bei einem sich geschwungen erstreckenden Knocheninnenraum. Bei derartigen Einsatzbedingungen erfordert die Scharfeinstellung des optischen Systems aufgrund der ständig wechselnden Entfernungen zum abzubildenden Objekt einen nicht unerheblichen Zeitbedarf, zum anderen wird dem Operateur durch ständig wechselnde Entfernungen des optischen Sensors zur Knochenwandung und die zeitweise Bildunschärfe die Orientierung innerhalb des Knocheninnenraumes erschwert.

Weitere Aufgabe der vorliegenden Erfindung ist es daher, eine Vorrichtung der einleitend genannten Art so zu verbessern, daß während der überwiegenden Einsatzdauer des Endoskops dem mit der Erzeugung der bildlichen Darstellung befaßten Operateuer eine einfache Orientierung der im Knocheninnenraum ermöglicht wird.

Diese Aufgabe wird erfindungsgemäß dadurch gelöst, daß im Bereich der dem Knocheninnenraum zugewandt angeordneten Ausdehnung des Endoskops eine die Qualität der bildlichen Darstellung verbessernde Ausstattung angeordnet ist.

Durch diese Ausstattung ist es möglich auch bei einer Bewegung des Endoskops relativ zu den den Knocheninnenraum begrenzenden Wandungen bildliche Darstellungen hoher Qualität zu erzeugen. Hierdurch ist es möglich, auch bei sich ändernden Bildausschnitten in kurzer zeitlicher Reihenfolge eine große Anzahl von bildlichen Darstellungen zu erzeugen.

Gemäß einer bevorzugten Ausführungsform der Erfindung ist die Ausstattung als Spreizvorrichtung ausgebildet. Die Spreizvorrichtung justiert das Endoskop quer zu seiner Längsachse gegenüber den Wandungen des Knocheninnenraumes. Das Endoskop kann im Bereich der Spreizvorrichtung fest gehaltert sein, es ist aber auch möglich, daß das Endoskop im Bereich der Spreizvorrichtung beweglich geführt ist. Durch die Spreizvorrichtung wird ein etwa konstanter Abstand des Endoskops zur Knochenwandung gewährleistet. Dieser etwa konstante Abstand ist insbesondere dann vorteilhaft, wenn das Endoskop in Richtung seiner Längsachse bewegt wird und bildliche Darstellungen von in Längsrichtung des Endoskops hintereinander liegenden Bereichen des Knocheninnenraums erzeugt werden. Der bei dieser Längsbewegung etwa konstante Abstand zur Knocheninnenwandung ermöglicht einem das Endoskop handhabenden Operateur die Orientierung im Knocheninnenraum.

Gemäß einer anderen bevorzugten Ausführungsform der Erfindung weist das Endoskop eine Autofocussiervorrichtung auf. Die Autofocussiervorrichtung ermöglicht auch bei einer Bewegung des Endoskops in Relation zur Wandung des Knocheninnenraums eine kurzfristige Scharfeinstellung der bildlichen Darstellung, ohne daß manuelle Eingriffe des die bildliche Darstellung erzeugenden Operateurs erforderlich werden. Der Operateur wird dadurch von Tätigkeiten entlaßtet, die seine Aufmerksamkeit von im Knocheninnenraum durchzuführenden Arbeiten ablenken könnten. Darüber hinaus ist es möglich, durch die nahezu kontinuierliche Bereitstellung einer scharfen bildlichen Darstellung kurzfristig Bearbeitungsvorgänge im Knocheninnenraum zu beenden oder ergänzende Tätigkeiten durchzuführen.

Weitere Einzelheiten der Erfindung ergeben sich aus der nachfolgenden Beschreibung und den beigefügten Zeichnungen, in denen bevorzugte Ausführungsformen von Vorrichtungen zur Durchführung des erfindungsgemäßen Verfahrens beispielsweise veranschaulicht sind.

In den Zeichnungen zeigen :

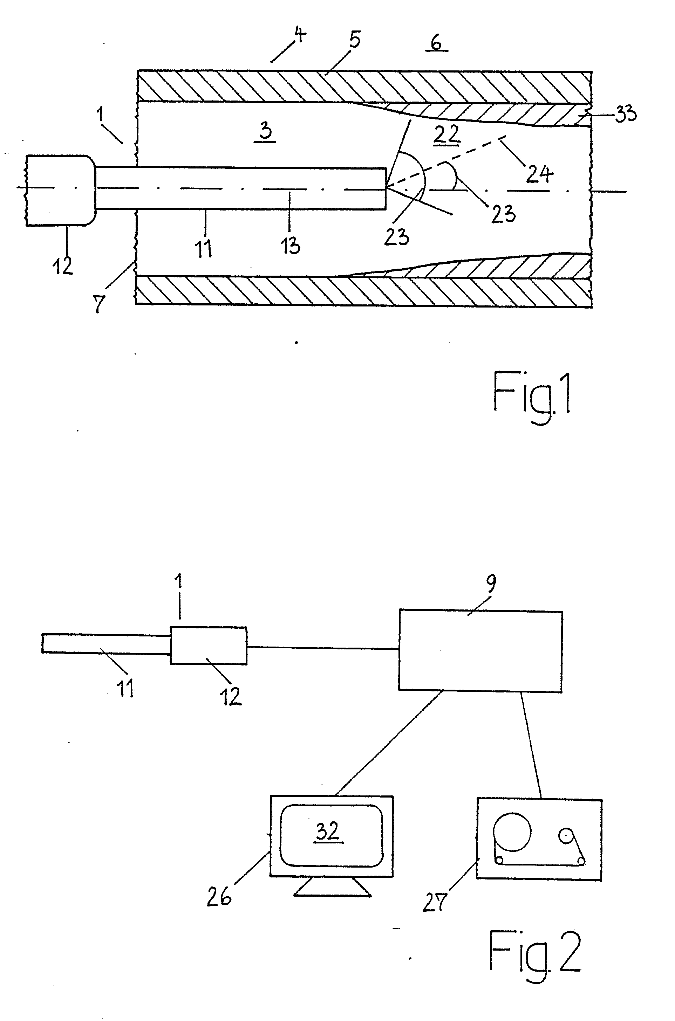

Figur 1: einen Längsschnitt durch einen Knochen, in dessen Knocheninnenraum ein Endoskop eingeführt ist,

Figur 2: eine prinzipielle Darstellung einer Vor richtung zur bildlichen Darstellung eines Knocheninnenraumes, die eine Zentraleinheit aufweist, die mit einem Endoskop, einem Bildschirm sowie einem Datenspeicher verbunden ist,

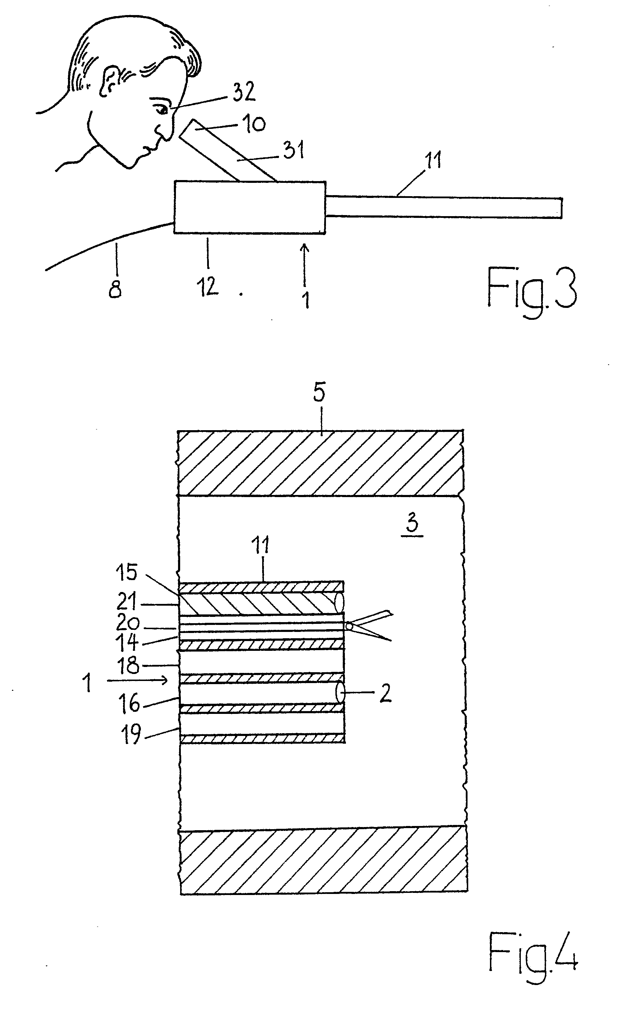

Figur 3: eine Seitenansicht eines Endoskops mit seitlichem Tubus,

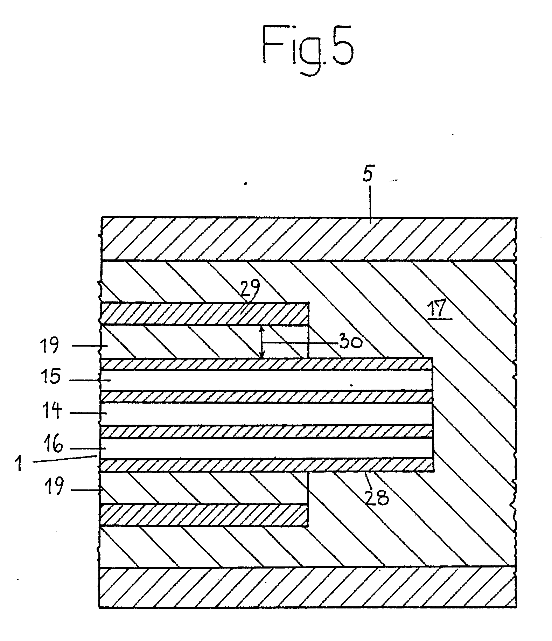

Figur 4: einen teilweise Darstellung eines Längsschnittes durch einen Knochen, in dessen Innenraum ein Endoskop eingeführt ist, das einen Instrumentenkanal, einen Lichtkanal, einen Zuführkanal sowie einen Ableitkanal aufweist,

Figur 5: eine teilweise Darstellung eines Längsschnittes durch ein Endoskop, das mindestens im Bereich seiner dem Knocheninnenraum zugewandten Ausdehnung doppelwandig ausgebildet ist und mit seiner Innenwandung und seiner Außenwandung einen Ableitkanal begrenzt,

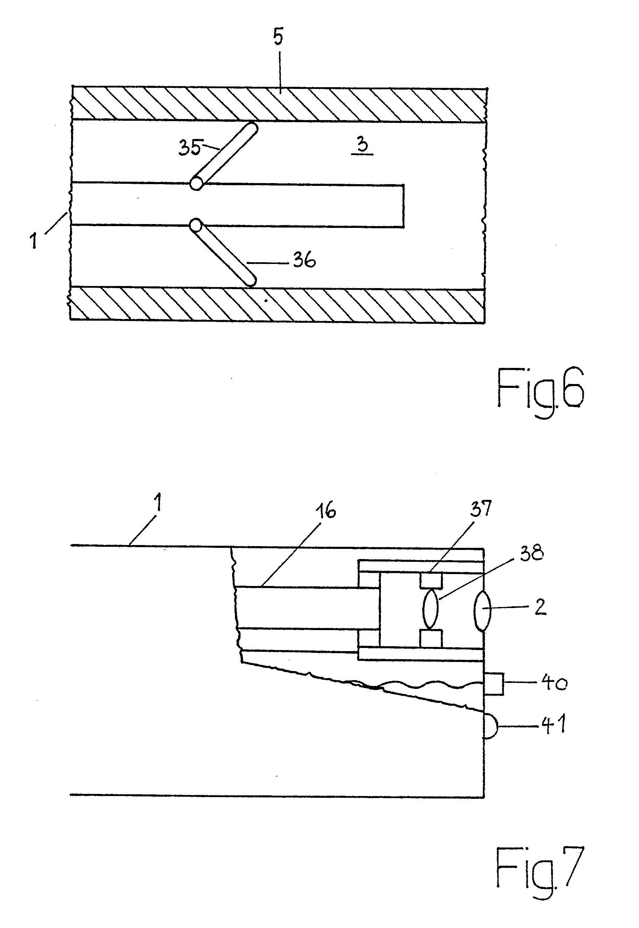

Figur 6: eine teilweise Darstellung eines Längsschnittes durch einen Knochen, in dessen Innenraum ein Endoskop eingeführt ist, das durch eine Spreizvorrichtung gegenüber den Wandungen des Knocheninnenraumes justiert ist,

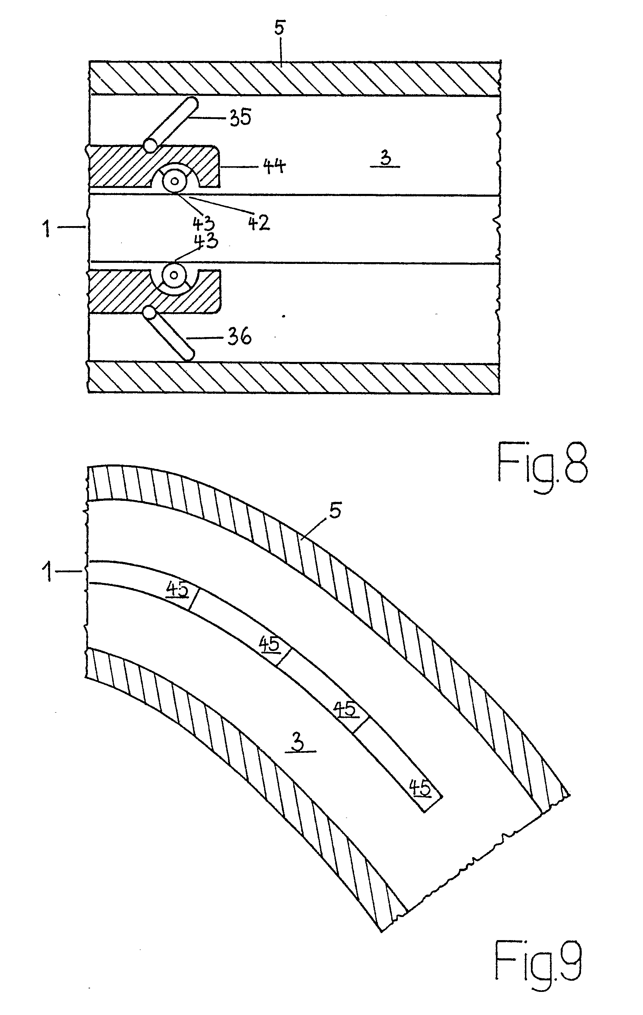

Figur 7: eine teilweise Darstellung eines Längsschnittes durch ein Endoskop, das eine Autofocussiervorrichtung aufweist,

Figur 8: eine teilweise Darstellung eines Längsschnittes durch einen Knochen, in dessen Innenraum ein Endoskop eingeführt ist, das einen aus Rollen ausgebildeten Endoskopantrieb aufweist und

Figure 9: eine teilweise Darstellung eines Längsschnittes durch einen geschwungen verlaufenden Knocheninnenraum, in den ein Endoskop eingeführt ist, das aus verschwenkbar miteinander verbundenen Endoskopgliedern ausgebildet ist.

Eine Vorrichtung zur Durchführung des erfindungsgemäßen Verfahrens besteht im wesentlichen aus einem als Endoskop (1) ausgebildeten optischen Sensor (2). Das Endoskop (1) ist in einem Knocheninnenraum (3) eines Knochens (4) angeordnet. Der Knocheninnenraum (3) ist von einer Knochenwandung (5) begrenzt, die in einem dem Endoskop (1) zugewandten Bereich ihrer Ausdehnung eine den Knocheninnenraum (3) mit einer Umgebung (6) verbindende Einführöffnung (7) aufweist. Diese wird entweder für die Einführung des Sensors (2) ausgebildet, oder eine bereits im Knochen (4) bestehende Öffnung wird als Einführöffnung (7) benutzt.

Das Endoskop (1) ist über eine Signalleitung (8) mit einer das Endoskop (1) versorgenden Zentraleinheit (9) verbunden. Es ist aber auch möglich, das Endoskop (1) als eigenständige Vorrichtung auszubilden, die ein eine unmittelbare visuelle Betrachtung ermöglichendes Okular (10) aufweist.

Das Endoskop (1) ist im wesentlichen als zylindrischer Schaft (11) ausgebildet, der im Bereich seines einen Endes in einen Handgriff (12) einmündet. Der Schaft (11) weist einen sich in Richtung einer Schaftlängsachse (13) erstrekkenden Instrumentenkanal (14), einen sich im wesentlichen parallel zum Instrumentenkanal (14) erstreckenden Lichtkanal (15) sowie einen Beobachtungskanal (16) auf. Es ist auch möglich, im Bereich des Schaftes einen Flüssigkeit (17) in den Bereich des Knocheninnenraumes (3) leitenden Zuführkanal (18) und einen Flüssigkeit (17) aus dem Knocheninnenraum (3) ableitenden Ableitkanal (19) vorzusehen. Durch den Instrumentenkanal (14) ist ein Bearbeitungsinstrument (20) in den Knocheninnenraum (3) hineingeführt. Im Bereich des dem Handgriff (12) abgewandte angeordneten Endes des Schaftes (11) ist im Beobachtungskanal (16) der optische Sensor (2) angeordnet. Im Bereich des Lichtkanals (15) ist ein Lichtleiter (21) angeordnet, der als Glasfaserkabel ausgebildet ist. Es ist aber auch möglich, den Lichtleiter (21) als im wesentlichen Flüssigkeit enthaltendes Fluidkabel auszubilden. Im Regelfall wird der Beobachtungskanal (16) auch als Lichtkanal (15) ausgebildet, durch den das Licht in den Knocheninnenraum (3) eingespiegelt wird.

Der optische Sensor (2) erfaßt einen Erfassungsraum (22), der im wesentlichen als ein Kegel ausgebildet ist, der mit seiner Spitze dem optischen Sensor (2) zugewandt ist und einen Erfassungswinkel (23) aufspannt. Der Erfassungsraum (22) weist eine sich durch den optischen Sensor hindurch (2) erstreckende Ausrichtungsmittellinie (24) auf, die mit der Schaftlängsachse (13) einen Ausrichtungswinkel (25) bildet.

Die Zentraleinheit (9) ist mit einem Sichtgerät (26) verbunden, auf dem das vom optischen Sensor (2) erfaßte Bild dargestellt ist. Die Zentraleinheit (9) ist gleichfalls mit einer Datenspeichervorrichtung (27) verbunden, die als elektronisches Speichermedium ausgebildet ist. Es ist aber auch möglich, die Datenspeichervorrichtung (27) als fotografische Vorrichtung auszubilden.

Der Ableitkanal (19) ist als Bohrung ausgebildet, die im Bereich des Schaftes (11) angeordnet ist. Es ist aber auch möglich, den Schaft (11) doppelwandig auszubilden und die Kanäle (14,15,16,18) im Bereich eines Innenkörpers (28) anzuordnen, der von einer im wesentlichen zylinderförmigen Außenwandung (29) umschlossen ist, die einen Abstand (30) zum Innenkörper (28) aufweist. Zwischen dem Innenkörper (28) und der Außenwandung (29) erstreckt sich der Ableitkanal (19).

Das Okular (10) eines für die unmittelbare visuelle Betrachung vorgesehenen Endoskopes (1) ist im Bereich dessen dem Schaft (11) abgewandt angeordneten Endes des Handgriffes (12) vorgesehen. Es ist aber auch möglich, das Okular (10) im Bereich eines dem Handgriff (12) abgewandt angeordneten Endes eines Tubus (31) anzuordnen, mit dem dieser in den Knocheninnenraum (3) hineinragt und der vom optischen Sensor (2) aufgenommene Signale in Richtung auf das Okular (10) leitet.

Bei einem visuellen Endoskop (1) ist eine Projektionsfläche (32), auf der die bildliche Darstellung des Knocheninnenraums abgebildet wird, als Netzhaut des menschlichen Auges ausgebildet. Ein mit einer Datenspeichereinrichtung (27) oder einem Sichtgerät (26) verbundenes Endoskop überträgt die Signale des optischen Sensors auf eine Projektionsfläche (32), die beispielsweise als Bildschirm oder als Filmnegativ ausgebildet ist.

Im Bereich des Endoskops (1) ist eine die Qualität der bildlichen Darstellung verbessernde Ausstattung (34) angeordnet. Die Ausstattung (34) ist als Spreizvorrichtung (35) ausgebildet, die mindestens eine das Endoskop gegenüber der Knochenwandung (5) verspannende Stütze (36) aufweist. Die Stütze (36) ist verschwenkbar im Bereich des Endoskops (1) gelagert. Es ist aber auch möglich, die Stütze (36) teleskopartig auszubilden. Die Spreizvorrichtung (35) weist ein handbetätigtes mechanisches Verstellgestänge auf. Es ist aber auch möglich, einen elektrischen Antrieb vorzusehen oder die Spreizvorrichtung (35) pneumatisch oder hydraulisch zu betätigen. Die Spreizvorrichtung (35) fixiert den optischen Sensor (2) in einem vorgegebenen Abstand zur Knochenwandung (5).

Im Bereich des optischen Sensors (2) ist eine Autofocussiervorrichtung (37) angeordnet. Die Autofocussiervorrichtung (37) ist mit einer Verstellvorrichtung verbunden, die ein von einem Empfänger (41) empfangenes Signal auswertet, das von einem im Bereich des dem Knocheninnenraum (3) zugewandten Endes des Endoskops angeordneten Sender (40) ausgesandt wird. Eine den Antrieb der Autofocussiervorrichtung steuernde Steuerung wertet die vom Empfänger (41) aufgenommenen Signale aus.

Das Endoskop (1) ist in einem Mantel (44) beweglich geführt, der einen Endoskopantrieb (42) aufweist, in dessen Bereich das Endoskop (1) bewegend beaufschlagende Rollen (43) angeordnet sind. Die Spreizvorrichtung (35) ist im Bereich des Mantels (44) angeordnet. Es ist aber auch möglich, die Spreizvorrichtung (35) im Bereich des innerhalb des Mantels (44) geführten Schaftes (11) des Endoskops (1) vorzusehen. Der Schaft (11) des Endoskops (1) ist aus einem elastischen Material ausgebildet. Es ist aber auch möglich, den Schaft (11) aus verschwenkbar miteinander verbundenen Endoskopgliedern (45) auszubilden.

Zur Erzeugung einer bildlichen Darstellung eines Knocheninnenraumes wird zunächst im Bereich des Knochens (4) eine Einführöffnung (7) erzeugt. Dies kann beispielsweise während einer Operation geschehen, bei der Teile des Knochens entfernt werden und dadurch ein Zugang zum Knocheninnenraum (3) geschaffen wird. Es ist aber auch möglich, die Einführöffnung bei der Implantation von Endoprothesen zu erzeugen und Bereiche des Knocheninnenraumes (13) abzubilden, die mindestens teilweise mit Knochenzement (33) gefüllt sind.

Durch die Einführöffnung (7) hindurch wird das Endoskop (1) in den Knocheninnenraum (3) eingeführt und mit Hilfe des optischen Sensors (2) werden Bereiche des Knocheninnenraumes (3) bildlich erfaßt. Zu diesem Zwecke kann das Endoskop (1) flexibel ausgestaltet sein, damit alle Teile des Knocheninnenraumes (3) auch bei einer leicht gekrümmten Gestaltung des Knochens (4) ausgeleuchtet und beobachtet werden können. Der optische Sensor (2) leitet die bei der Bilderfassung erzeugten Signale in Richtung auf die Projektionsvorrichtung (32) weiter. Im Bereich der Projektionsvorrichtung (32) wird die bildliche Darstellung des Knocheninnenraumes (3) erzeugt.

Zur Erzeugung einer bildlichen Darstellung hoher Qualität wird durch den Lichtkanal (15) Licht in den Knocheninnenraum (3) eingeleitet und dadurch eine ausreichende Bildhelligkeit erzeugt. Gleichfalls wird durch den Ableitkanal trübe, den Knocheninnenraum (3) ausfüllende, Flüssigkeit (17) entfernt und durch den Zuführkanal (18) durch saubere Flüssigkeit (17) ersetzt. Durch diesen Flüssigkeitsaustausch wird auch bei Blutungen innerhalb des Knocheninnenraumes (3) oder bei der Ablösung von Teilen der Knochenwandung (5) bzw. der Bearbeitung von Knochenzement (33) stets für eine ausreichende Bildklarheit gesorgt, so daß auch Defekte beziehungsweise Veränderungen der Knochenwandungen (5) beobachtet werden können.

Bei einer bildlichen Darstellung des Knocheninnenraumes zu unterschiedlichen Zeitpunkten während einer Operation können mit Hilfe der Datenspeichereinrichtung (27) eine beliebige Anzahl von bildlichen Darstellungen abgespeichert und einer späteren Weiterverarbeitung zugeführt werden.

**Ansprüche**

1. Verfahren zur Erzeugung einer bildlichen Darstellung eines Knocheninnenraumes, dadurch gekennzeichnet, daß im Bereich einer den Knocheninnenraum (3) begrenzendenden Knochenwandung (5) eine Einführöffnung (7) erzeugt und anschließend durch die Einführöffnung (7) hindurch ein optische Signale zu einer Projektionsvorrichtung (32) übertragender vorgebbar positionierbarer optischer Sensor (2) in den Knocheninnenraum (3) eingeführt wird.

2. Verfahren nach Anspruch 1, dadurch gekennzeichnet, daß durch die Einführöffnung (7) hindurch ein Endoskop (1) eingeführt wird.

3. Verfahren nach Einspruch 1 und 2, dadurch gekennzeichnet, daß der Knocheninnenraum (3) beleuchtet wird.

4. Verfahren nach Anspruch 3, dadurch gekennzeichnet, daß der Knocheninnenraum (3) mit Kaltlicht beleuchtet wird.

5. Verfahren nach Anspruch 1 bis 4, dadurch gekennzeichnet, daß das Licht über Glasfasern in den Bereich des Knocheninnenraumes (3) geleitet wird.

6. Verfahren nach Anspruch 1 bis 4, dadurch gekennzeichnet, daß das Licht durch ein als Flüssigkeitslichtkabel ausgebildetes Fluidkabel in den Bereich des Knocheninnenraumes (3) geleitet wird.

7. Verfahren nach Anspruch 1 bis 6, dadurch gekennzeichnet, daß die vom optischen Sensor (2) erzeugten optischen Signale auf eine als menschliche Netzhaut ausgebildete Projektionsvorrichtung (32) übertragen werden.

8. Verfahren nach Anspruch 1 bis 6, dadurch gekennzeichnet, daß die optischen Signale auf eine anorganische Projektionsvorrichtung übertragen werden.

9. Verfahren nach Anspruch 8, dadurch gekennzeichnet, daß die optischen Signale auf einen Bildschirm übertragen werden.

10. Verfahren nach Anspruch 1 bis 9, dadurch gekennzeichnet, daß die optischen Signale mindestens teilweise gespeichert werden.

11. Verfahren nach Anspruch 10, dadurch gekennzeichnet, daß die optischen Signale fotografisch gespeichert werden.

12. Verfahren nach Anspruch 10, dadurch gekennzeichnet, daß die optischen Signale elektronisch gespeichert werden.

13. Verfahren nach Anspruch 1 bis 12, dadurch gekennzeichnet, daß in den Knocheninnenraum (3) eine seine optische Erfaßbarkeit verbessernde Flüssigkeit (17) eingeleitet wird.

14. Verfahren nach Anspruch 13, dadurch gekennzeichnet, daß verbrauchte Flüssigkeit (17) aus dem Knocheninnenraum (3) abgeleitet wird.

15. Verfahren nach Anspruch 1 bis 14, dadurch gekennzeichnet, daß die Knochenwandung (5) einer die bildliche Darstellung verändernden Bearbeitung unterworfen wird.

16. Verfahren nach Anspruch 15, dadurch gekennzeichnet, daß während der Bearbeitung Knochenzement (33) von der Knochenwandung (5) abgetrennt wird.

17. Verfahren nach Anspruch 16, dadurch gekennzeichnet, daß der Knochenzement (33) mechanisch entfernt wird.

18. Verfahren nach Anspruch 17, dadurch gekennzeichnet, daß der Knochenzement (33) mindestens bereichsweise durch Hämmern entfernt wird.

19. Verfahren nach Anspruch 17, dadurch gekennzeichnet, daß der Knochenzement (33) mindestens bereichsweise durch Stoßen entfernt wird.

20. Verfahren nach Anspruch 16, dadurch gekennzeichnet, daß der Knochenzement (33) mit hochenergetischer Lichtstrahlung entfernt wird.

21. Verfahren nach Anspruch 16, dadurch gekennzeichnet, daß der Knochenzement (33) mit Ultraschall entfernt wird.

22. Verfahren nach Anspruch 1 bis 21, dadurch gekennzeichnet, daß eine einen Erfassungsraum (22) im wesentlichen zentrisch durchdringende Ausrichtungsmittellinie (24) des optischen Sensors (2) gegenüber einer Schaftlängsachse (13) des Endoskops (1) verschwenkt wird.

23. Verfahren nach Anspruch 1 bis 22, dadurch gekennzeichnet, daß ein flexibler Schaft (11) des Endoskopes (1) entsprechend einer individuellen Ausgestaltung des Knocheninnenraumes (3) verformt wird.

24. Verfahren nach Anspruch 1 bis 23, dadurch gekennzeichnet, daß im Bereich des optischen Sensors (2) eine mindestens bereichsweise in Richtung auf einen Handgriff (12) des Endoskops (1) ausgerichtete Strömung von Flüssigkeit (17) erzeugt wird.

25. Verfahren nach Anspruch 1 bis 24, dadurch gekennzeichnet, daß der optische Sensor (2) zur Beobachtung eines speziellen Ausschnittes der Knochenwandung (5) auf diesen scharf eingestellt wird.

26. Vorrichtung zur Erzeugung einer bildlichen Darstellung eines Knocheninnenraumes, dadurch gekennzeichnet, daß im Bereich der dem Knocheninnenraum (3) zugewandt angeordneten Ausdehnung des Endoskops (1) eine die Qualität der bildlichen Darstellung verbessernde Ausstattung (34) angeordnet ist.

27. Vorrichtung nach Anspruch 26, dadurch gekennzeichnet, daß die Ausstattung (34) als Spreizvorrichtung (35) ausgebildet ist, die mindestens eine im Bereich des Endoskops (1) angeordnete Stütze (36) aufweist.

28. Vorrichtung nach Anspruch 27, dadurch gekennzeichnet, daß die Stütze (36) im Bereich des Endoskops (1) schwenkbar gelagert ist.

29. Vorrichtung nach Anspruch 27, dadurch gekennzeichnet, daß die Sütze (36) teleskopierbar ausgebildet ist.

30. Vorrichtung nach Anspruch 26 bis 29, dadurch gekennzeichnet, daß die Spreizvorrichtung (35) einen mechanischen Antrieb aufweist.

31. Vorrichtung nach Anspruch 30, dadurch gekennzeichnet, daß der Antrieb handbetätigt ausgebildet ist.

32. Vorrichtung nach Anspruch 30, dadurch gekennzeichnet, daß der Antrieb elektrisch ausgebildet ist.

33. Vorrichtung nach Anspruch 30, dadurch gekennzeichnet, daß der Antrieb im wesentlichen pneumatisch ausgebildet ist.

34. Vorrichtung nach Anspruch 30, dadurch gekennzeichnet, daß der Antrieb im wesentlichen hydraulisch ausgebildet ist.

35. Vorrichtung nach Anspruch 26 bis 34, dadurch gekennzeichnet, daß das Endoskop (1) eine Autofocussiervorrichtung (37) aufweist.

36. Vorrichtung nach Anspruch 26 bis 35, dadurch gekennzeichnet, daß im Bereich des Endoskops (1) ein ein Meßsignal abstrahlender Sender (40) und ein ein reflektiertes Meßsignal empfangender Empfänger (41) angeordnet ist, der mit einer die Autofocussiervorrichtung (37) steuernden Steuerung verbunden ist.

37. Vorrichtung nach Anspruch 36, dadurch gekennzeichnet, daß die Steuerung mit einem die Autofocussiervorrichtung (37) verstellenden Antrieb verbunden ist.

38. Vorrichtung nach Anspruch 37, dadurch gekennzeichnet, daß der Antrieb im Bereich des Endoskops (1) angeordnet ist.

39. Vorrichtung nach Anspruch 37, dadurch gekennzeichnet, daß der Antrieb außerhalb des Endoskops (1) angeordnet ist.

40. Vorrichtung nach Anspruch 26 bis 39, dadurch gekennzeichnet, daß im Bereich des Endoskops (1) ein es bewegender Endoskopantrieb (42) angeordnet ist.

41. Vorrichtung nach Anspruch 40, dadurch gekennzeichnet, daß das Endoskop (1) von mindestens einer es antreibenden Rolle (43) beaufschlagt ist, die im Bereich eines das Endoskop (1) mindestens bereichsweise umschließenden Mantels (44) angeordnet ist.

42. Vorrichtung nach Anspruch 40, dadurch gekennzeichnet, daß das Endoskop (1) teleskopierbar ausgebildet ist.

43. Vorrichtung nach Anspruch 26 bis 42, dadurch gekennzeichnet, daß die Spreizvorrichtung (35) im Bereich des Mantels (44) angeordnet ist.

44. Vorrichtung nach Anspruch 26 bis 43, dadurch gekennzeichnet, daß das Endoskop (1) mindestens bereichsweise entlang der Schaftlängsachse (13) flexibel ausgebildet ist.

45. Vorrichtung nach Anspruch 44, dadurch gekennzeichnet, daß das Endoskop (1) aus verschwenkbar miteinander verbundenen Endoskopgliedern (45) ausgebildet ist.

Fig.1

Fig.2

Fig.3

Fig.4

# Fig.5

Fig.6

Fig.7

Fig.8

Fig.9